# EUROPÄISCHE PATENTSCHRIFT

(11) **EP 0 734 701 B1**
(45) Veröffentlichungstag und Bekanntmachung des Hinweises auf die Patenterteilung: **11.10.2000**
(21) Anmeldenummer: 96108125.4
(22) Anmeldetag: 08.12.1992
(51) Int. Cl.: A61F 2/30, A61F 2/38

(54) **Künstliches Gelenk**
Artificial joint
Articulation artificielle

(30) Priorität: 11.12.1991 DE 4140837; 31.01.1992 DE 4202717
(43) Veröffentlichungstag der Anmeldung: 02.10.1996
(62) Teilanmeldung aus: 92924687.4
(73) Patentinhaber: HJS Gelenk System GmbH, 81925 München (DE)
(72) Erfinder: Kubein-Meesenburg, Dietmar, Prof. Dr., 37547 Kreiensen (DE); Nägerl, Hans, Prof. Dr., 37130 Gleichen (DE)
(74) Vertreter: Braun, Dieter, Dipl.-Ing.

(56) Entgegenhaltungen:
- EP-A- 0 442 330
- WO-A-87/04917
- DE-A- 3 908 958

## Beschreibung

Die vorliegende Erfindung betrifft ein künstliches Gelenk nach dem Oberbegriff des Anspruchs 1.

Aus der deutschen Patentanmeldung P 39 08 958.4 ist bereits ein künstliches Gelenk bekannt, zum Ersatz insbesondere von menschlichen Gelenken, bestehend aus mindestens zwei Gelenkteilen mit zueinander sich bewegenden sphärischen Funktionsflächen. Die Krümmungsverhältnisse der eine kreisförmige Schnittkontur aufweisenden Funktionsflächen sind zueinander konvex-konvex, konvex-konkav oder konkav-konkav, und die Gelenkgeometrie ist durch eine Gelenkkette mit zwei Gelenkachsen (dimere Gelenkkette) bestimmt, die durch die Rotationszentren der Funktionsflächen verlaufen. Hierbei sind die Gelenkflächen kugelförmig ausgebildet, so daß eine Gelenkbewegung mit fünf Freiheitsgraden möglich ist.

Es hat sich jedoch gezeigt, daß derartige Gelenke nicht geeignet sind, um spezielle Gelenkfunktionen des menschlichen Kniegelenks nachzubilden.

Aus der europäischen Patentanmeldung 0 442 330 A2 ist ein künstliches Gelenk nach dem Oberbegriff des Anspruchs 1 bekannt. Nachteilig bei diesem Gelenk ist jedoch die geringe stabilität in einer zur Längsebene um 90° versetzten Querebene.

Der Erfindung liegt die Aufgabe zugrunde, ein künstliches Gelenk zu schaffen, das eine Bewegungsfreiheit nur in einer Gelenkebene besitzt und das gleichzeitig eine hohe mechanische Stabilität mit einer großen Variationsbreite zur Anpassung an individuelle Gegebenheiten aufweist.

Erfindungsgemäß wird dies durch ein künstliches Gelenk gemäß den Merkmalen des Anspruchs 1 erreicht. Durch die erfindungsgemäß vorgesehenen Gelenkflächen, die Flächensegmente eines toroidförmigen Körpers sind, ergibt sich eine Bewegungsfreiheit nur in einer der Ebenen, und zwar in der Längsebene, wohingegen in der hierzu senkrechten Querebene eine eingeschränkte Bewegungsfreiheit vorhanden ist. Die auftretenden Druckbeanspruchungen können durch die Verwendung entsprechend fester Materialien beherrscht werden. Der vorteilhafterweise vorhandene Druckverteilungskörper gewährleistet eine druckkraftschlüssige Bindung zwischen den beiden Gelenkteilen und eine Verteilung der auftretenden Druckbelastungen, auf eine größere Fläche, woraus sich eine hohe mechanische Stabilität ergibt und ermöglicht wird, Materialien zu verwenden, die eine geringere mechanische Druckfestigkeit besitzen.

Das erfindungsgemäße Gelenk weist in der sagittalen Ebene (Längsebene) eine nicht überschlagene druckkraftschlüssige dimere Gelenkkette auf, die wegen ihrer Ausgestaltung der Gelenkflächen eine eingeschränkte Bewegungsfreiheit in der frontalen Ebene (Querebene) besitzt. Bei der erfindungsgemäßen Ausgestaltung müssen die Rotationszentren M₈₁ und M₉₁ nicht zusammenfallen. Das Rotationszentrum M₉₁ kann in Richtung auf den Oberschenkel und/oder seitlich nach innen (medial) oder außen (lateral) versetzt von dem Rotationszentrum M₈₁ liegen.

Weitere vorteilhafte Eigenschaften sind in den Unteransprüchen enthalten.

Anhand der in den beiliegenden Zeichnungen enthaltenen Ausführungsbeispiele wird die Erfindung näher erläutert. Es zeigen:
- Fig. 1: einen Schnitt in der sagittalen Ebene (Längsebene) durch eine Ausführung eines erfindungsgemäßen Gelenks mit Druckverteilungskörper,
- Fig. 2: einen Schnitt durch das Gelenk gemäß Fig. 1, jedoch in der frontalen Ebene, d.h. in einer um 90° gedrehten Querebene zu Fig. 1 und
- Fig. 3 und 4: Ansichten gemäß den Figuren 1 und 2 durch ein erfindungsgemäßes Gelenk ohne Druckverteilungskörper.

Ein menschliches Kniegelenk besteht aus zwei Gelenkteilen, und zwar dem medialen Gelenkteil und dem lateralen Gelenkteil.

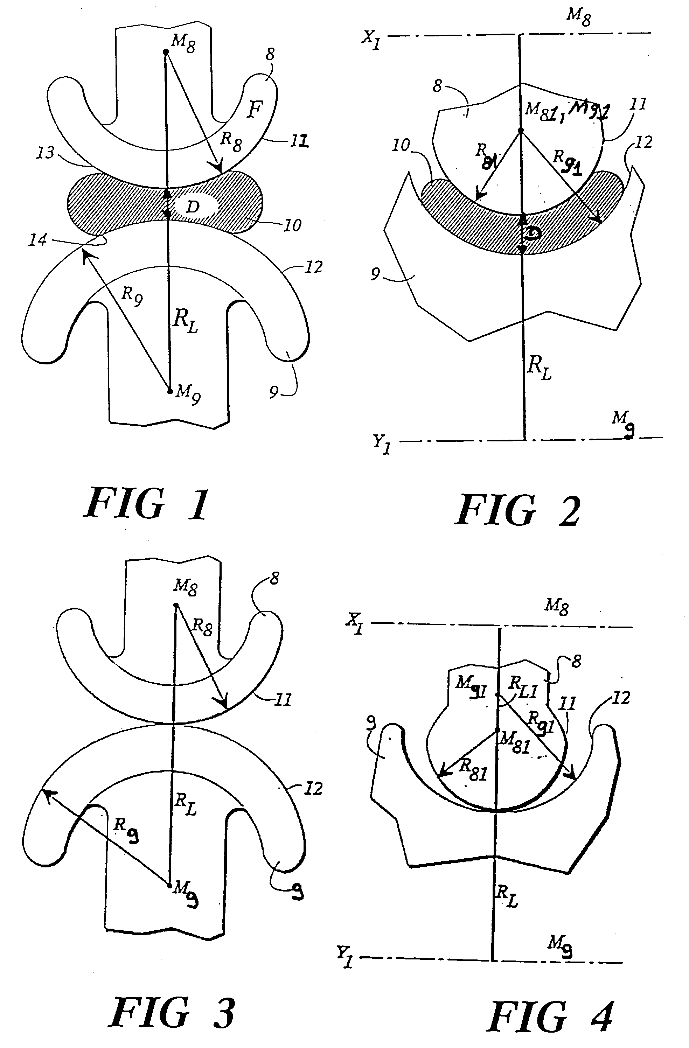

In Fig. 1 ist als Schnitt durch die Längsebene bzw. in der sagittalen Ebene eines erfindungsgemäßen Gelenks dargestellt, das den lateralen Gelenkteil eines menschlichen Kniegelenks bilden kann. Der laterale Gelenkteil ersetzt die natürliche Artikulation zwischen dem lateralen femoralen und lateralen tibialen Kondylus. Das Gelenk gemäß Fig. 1 besteht aus den Gelenkteilen 8, 9, und zwar einem Gelenkkopf 8 und einer Gelenkpfanne 9, zwischen denen ein Druckverteilungskörper 10 beweglich angeordnet ist. Das Gelenkteil 8 stellt dabei das Femurteil dar, das starr mit dem Knochen des lateralen femoralen Kondylus verbunden wird im menschlichen Körper, und das Gelenkteil 9 stellt das Tibiateil dar, das starr mit dem Knochen des lateralen tibialen Kondylus im menschlichen Körper verbunden wird. Die von den Gelenkteilen 8, 9 gebildeten Gelenkflächen 11, 12 sind als Flächensegmente eines toroidförmigen Körpers ausgebildet, so daß die den Gelenkflächen 11, 12 zugekehrten Gleitflächen 13, 14 des Diskus 10 ebenfalls in Anpassung an die Ausgestaltung der Gelenkflächen 11, 12 toroidförmig ausgestaltet sind. Das Gelenkteil 8 weist in Fig. 1 in seiner Längsebene ebenfalls eine Funktionsfläche mit einer kreisförmigen Schnittkontur auf, wobei diese kreisförmige Schnittkontur den Mittelpunkt bzw. das Rotationszentrum M₈ und den Radius R₈ besitzt, so daß sich ein konvexer Verlauf der Funktionsfläche ergibt. Das Gelenkteil 9 weist den Mittelpunkt M₉ bzw. das Rotationszentrum M₉ auf und besitzt den Radius R₉, so daß sich ebenfalls eine konvexe Form der kreisbogenförmigen Schnittkontur der Funktionsfläche ergibt. Wie dargestellt ist, liegen die Rotationszentren M₈ und M₉ jeweils im zugehörigen Gelenkteil 8 und 9. Die Gelenkachsenbahn der Rotationszentren M₈ und M₉ besitzt einen Radius R_{L} = R₈ + R₉ + D , wobei D die minimale Dicke des Diskus 10 auf der Verbindung zwischen den beiden Rotationszentren M₈ und M₉ ist.

In Fig. 2 ist der Schnitt gemäß der Frontalebene, Querebene, zu der Darstellung in Fig. 1 dargestellt. Hierbei ist zu erkennen, daß auch in dieser Schnittebene die Gelenkkörper 8, 9 jeweils kreisförmige Schnittkonturen ihrer Funktionsflächen besitzen. Der Gelenkkörper 8 weist dabei eine kreisförmige Schnittkontur mit dem Mittelpunkt M₈₁ und dem Radius R₈₁ auf, wobei eine konvexe Funktionsfläche gebildet wird. Der Gelenkkörper 9 besitzt den Mittelpunkt M₉₁ und den Radius R₉₁, wobei eine konkave Ausbildung der kreisförmigen Schnittkontur besteht. Hierbei liegen die Rotationszentren bzw. die Mittelpunkte M₈₁ und M₉₁ innerhalb des Gelenkteils 8 mit der konvexen Schnittkontur der Funktionsfläche, und die Gelenkachsenbahn der Rotationszentren M₈₁, M₉₁ ist im dargestellten Ausführungsbeispiel aufeinanderfallend angeordnet. Die Rotationszentren M₈₁ und M₉₁ müssen ebenfalls nicht zusammenfallen. Das Rotationszentrum M₉₁ kann in Richtung auf den Oberschenkel und/oder seitlich nach innen (medial) oder außen (lateral) versetzt von dem Rotationszentrum M₈₁ liegen. Weiterhin ist zu erkennen, daß die Rotationszentren M₈₁ und M₉₁ nicht mit den Rotationsachsen X₁, Y₁ durch die Rotationszentren M₈ und M₉ zusammenfallen. Die kreisbogenförmigen Funktionsflächen in Fig. 1 stellen eine nicht überschlagene druckkraftschlüssige dimere Gelenkkette dar, wobei wegen der toroidförmigen Ausformung der Gelenkflächen keine Bewegungsfreiheit in der frontalen Ebene, siehe Fig. 2, gegeben ist.

Wesentlich für die einwandfreie Funktion des erfindungsgemäßen Gelenks gemäß den Figuren 1 und 2 ist, daß der Druckverteilungskörper 10 beweglich ist. Hierzu müßten die Gleitflächen 13, 14 zwischen dem Druckverteilungskörper und den Gelenkteilen 8, 9 eine möglichst geringe Reibung zu den Gelenkteilen besitzen, und zudem muß die Reibungskraft zwischen den anliegenden Flächen beidseitig des Druckverteilungskörpers jeweils gleich groß sein. Dies wird erfindungsgemäß dadurch erreicht, daß die wirksamen Gleitflächen 13, 14 des Druckverteilungskörpers 10 zu den jeweils angrenzenden Gelenkteilen 8, 9 gleich groß ausgelegt werden. Die Größe der jeweiligen Kontaktfläche kann durch die Wahl der toroidalen Wulstradien sowie durch die Gestaltung der Randwülste des Druckverteilungskörpers erreicht werden. Zudem sind die Gleitflächen 13, 14 hochglanzpoliert.

In den Figuren 3 und 4 ist ein dem Gelenk gemäß den Figuren 1 und 2 entsprechendes Gelenk jedoch ohne Druckverteilungskörper dargestellt. Hierbei sind gleiche Teile jeweils mit denselben Bezugsziffern versehen. Hinsichtlich der geometrischen Beziehungen der jeweiligen Radien zueinander gilt hier die Bedingung D = 0. Geht man hierbei davon aus, daß der Radius R_{L} bei beiden Gelenken gemäß den Figuren 1, 2 und den Figuren 3 und 4 gleich groß ist, so wird man zweckmäßigerweise R₈ und R₈₁ gemäß den Figuren 3 und 4 größer wählen als beim Gelenk gemäß Figuren 1, 2. Durch diese Maßnahme wird die Berührungsfläche zwischen den Gelenkflächen 11, 12 möglichst groß.

## Patentansprüche

1. Künstliches Gelenk als Endoprothese für ein laterales Gelenkteil eines menschlichen Kniegelenks, bestehend aus einem Gelenkkopf (8) und einer Gelenkpfanne (9), wobei der Gelenkkopf (8) das Femurteil und die Gelenkpfanne (9) das Tibiateil bildet, wobei der Gelenkkopf (8) und die Gelenkpfanne (9) Gelenkflächen (11, 12) aufweisen, die Flächensegmente eines toroidförmigen Körpers sind, und der Gelenkkopf (8) und die Gelenkpfanne (9) Funktionsflächen mit kreisförmigen Schnittkonturen mit den Radien R₈, R₉ besitzen und die Gelenkgeometrie der Funktionsflächen zueinander in einer Längsebene durch eine Gelenkkette mit zwei Gelenkachsen bestimmt ist, die durch die Rotationszentren M₈, M₉ der Funktionsflächen mit den Radien R₈, R₉ der Schnittkonturen in der Längsebene verlaufen, wobei die Schnittkonturen in der Längsebene konvex-konvex derart ausgebildet sind, daß ihre Rotationszentren M₈, M₉ in dem jeweils zugehörigen Gelenkteil (8, 9) liegen und die Gelenkachsenbahn der Rotationszentren M₈, M₉ einen Radius R_{L} = R₈ + R₉ besitzt, dadurch gekennzeichnet, daß die Gelenkgeometrie der Funktionsflächen zueinander in einer zur Längsebene um 90 ° versetzten Querebene durch eine Gelenkkette mit zwei Gelenkachsen bestimmt ist, die durch die Rotationszentren M₈₁, M₉₁ der Funktionsflächen mit den Radien R₈₁, R₉₁ der in der Querebene liegenden kreisförmigen Schnittkonturen verlaufen, wobei deren Krümmungsverhältnisse derart konvex-konkav ausgebildet sind, daß ihre Rotationszentren M₈₁, M₉₁ innerhalb des Gelenkteils (8) mit der konvexen Schnittkontur der Funktionsfläche liegen und der Abstand der Rotationszentren R_{L1} = R₉₁ - R₈₁ ist, wobei R₉₁ ≥ R₈₁ ist, sowie die Rotationszentren M₈₁, M₉₁ nicht mit den Rotationsachsen (X₁/Y₁) durch die Rotationszentren M₈, M₉ zusammenfallen.

2. Künstliches Gelenk nach Anspruch 1, dadurch gekennzeichnet, daß ein Druckverteilungskörper (10) zwischen den Gelenkflächen (11, 12) angeordnet ist, dessen an den Gelenkflächen (11, 12) anliegende Gleitflächen (13, 14) eine den Gelenkflächen (11, 12) angepaßte Ausgestaltung der Flächensegmente eines toroidförmigen Körpers aufweisen, wobei der Druckverteilungskörper (10) eine minimale Dicke (D) besitzt, die auf der Verbindungslinie der Rotationszentren der Funktionsflächen liegt.

3. Künstliches Gelenk nach Anspruch 2, dadurch gekennzeichnet, daß in der Längsebene die Gelenkachsenbahn der Rotationszentren M₈, M₉ einen Radius R_{L} = R₈ + R₉ + D besitzt und in der Querebene der Abstand der Rotationszentren R_{L1} = R₉₁ - R₈₁ - D ist, wobei R₉₁ ≥ R₈₁ + D ist.

4. Künstliches Gelenk nach einem der Ansprüche 1 bis 3, dadurch gekennzeichnet, daß die Rotationszentren M₈₁, M₉₁ zusammenfallen oder derart auseinanderliegen, daß das Rotationszentrum M₉₁ in Richtung auf den Oberschenkel und/oder seitlich nach innen, medial, oder nach außen, lateral, versetzt von dem Rotationszentrum M₈₁ liegt.

5. Künstliches Gelenk nach einem der Ansprüche 1 bis 4, dadurch gekennzeichnet, daß die beiden Gleitflächen (13, 14) des Druckverteilungskörpers (10) gleich groß sind.

## Claims

1. An artificial joint as an endoprosthesis for a lateral joint part of a human knee joint, comprising a joint head (8) and a joint socket (9), whereby the joint head (8) forms the femur part and the joint socket (9) the tibia part, whereby the joint head (8) and the joint socket (9) have joint surfaces (11, 12), which are surface segments of a toroidal shaped body, and the joint head (8) and the joint socket (9) have functional surfaces with circular cross section contours with radii R₈, R₉ and the joint geometry of the functional surfaces to each other is determined in a longitudinal plane by a link chain with two hinge axes, which run through the centres of rotation M₈, M₉ of the functional surfaces with radii R₈, R₉ of the sectional contours in the longitudinal plane, whereby the sectional contours are formed convex-convex in the longitudinal plane in such a manner that their centres of rotation M₈, M₉ lie in their respective joint parts (8, 9) and the joint axis path of the centres of rotation M₈, M₉ possesses a radius of R_{L} = R₈ + R₉, characterised in that the joint geometry of the functional surfaces is determined in a cross plane displaced by 90° to the longitudinal axis by a link chain with two joint axes, which runs through the centres of rotation M₈₁, M₉₁ of the functional surfaces with the radii R₈₁, R₉₁ which run within the circular section sectional contours lying within the cross plane, whereby their curvature ratios are formed convex-concave such that their centres of rotation M₈₁, M₉₁ lie within the joint part (8) with the convex sectional contour of its functional surface and the separation of the centres of rotation is R_{L1} = R₉₁ - R₈₁, where R₉₁ ≥ R₈₁, and the centres of rotation M₈₁, M₉₁ do not come together through the centres of rotation M₈, M₉.

2. An artificial joint according to Claim 1, characterised in that a pressure distributing body (10) is arranged between the joint surfaces (11, 12), whose sliding surfaces (13, 14), lying on the joint surfaces (11, 12) have a toroid-shaped body matched to the joint surfaces (11, 12), whereby the pressure distributing body (10) has a minimum thickness (D), which lies on the line joining the centres of rotation of the functional surfaces.

3. An artificial joint according to Claim 2, characterised in that in the longitudinal plane the joint axis path of the centres of rotation M₈, M₉ has a radius R_{L} = R₈ + R₉ + D and in the cross plane the separation of the centres of rotation is R_{L1} = R₉₁ - R₈₁ - D, whereby R₉₁ ≥ R₈₁ + D.

4. An artificial joint according to one of the Claims 1 to 3, characterised in that the centres of rotation M₈₁, M₉₁ come together or are displaced from each other such that the centre of rotation M₉₁ in the direction of the thigh and/or sideways towards the inside, medial, or towards the outside lateral, lies displaced from the centre of rotation M₈₁.

5. An artificial joint according to one of the Claims 1 to 4, characterised in that the two sliding surfaces (13, 14) of the pressure distributing body (10) are of equal size.

## Revendications

1. Articulation artificielle servant d'endoprothése pour une partie articulaire latérale d'une articulation de genou humain, constituée d'un condyle (8) et d'une glène (9), le condyle (8) formant la partie fémorale et la glène (9) la partie tibiale, le condyle (8) et la glène (9) comportant des surfaces articulaires (11, 12) qui sont des segments de surface d'un corps toroïdal, et le condyle (8) et la glène (9) possédant des surfaces fonctionnelles avec des contours en coupe circulaires de rayons R₈, R₉, et la géométrie articulaire des surfaces fonctionnelles l'une par rapport à l'autre dans un plan longitudinal étant déterminée par une chaîne articulaire à deux axes articulaires qui passent par les centres de rotation M₈, M₉ des surfaces fonctionnelles de rayons R₈, R₉ des contours en coupe dans le plan longitudinal, les contours en coupe dans le plan longitudinal étant conçus sous forme convexe-convexe, de façon que leurs centres de rotation M₈, M₉ se trouvent dans chacune des parties articulaires correspondantes (8, 9) et que le trajet des axes articulaires entre les centres de rotation M₈, M₉ possède un rayon R_{L} = R₈ + R₉, caractérisée en ce que la géométrie articulaire des surfaces fonctionnelles l'une par rapport à l'autre dans un plan transversal tourné de 90° par rapport au plan longitudinal est déterminée par une chaîne articulaire à deux axes articulaires qui passent par les centres de rotation M₈₁, M₉₁ des surfaces fonctionnelles de rayons R₈₁, R₉₁ des contours en coupe circulaires situés dans le plan transversal, les rapports de courbure de ces derniers étant conçus sous forme convexe-concave, de façon que leurs centres de rotation M₈₁, M₉₁ se trouvent à l'intérieur de la partie articulaire (8) possédant le contour en coupe convexe de la surface fonctionnelle, et la distance entre les centres de rotation est R_{L1} = R₉₁-R₈₁, sachant que R₉₁ ≥ R₈₁, et les centres de rotation M₈₁, M₉₁ ne coïncidant pas avec les axes de rotation (X₁/Y₁) passant par les centres de rotation M₈, M₉.

2. Articulation artificielle selon la revendication 1 caractérisée en ce que, entre les surfaces articulaires (11,12), est disposé un corps de répartition de pression (10) dont les surfaces de glissement (13, 14) en contact avec les surfaces articulaires (11, 12) présentent la configuration, adaptée aux surfaces articulaires (11, 12), des segments de surface d'un corps toroidal, le corps de répartition de pression (10) possédant une épaisseur minimale (D) qui se trouve sur la ligne de jonction des centres de rotation des surfaces fonctionnelles.

3. Articulation artificielle selon la revendication 2, caractérisée en ce que le trajet des axes articulaires entre les centres de rotation M₈, M₉ possède un rayon R_{L} = R₈ + R₉ + D, et la distance entre les centres de rotation est R_{L1} = R₉₁ - R₈₁ - D, sachant que R₉₁ ≥ R₈₁ + D.

4. Articulation artificielle selon l'une des revendications 1 à 3, caractérisée en ce que les centres de rotation M₈₁, M₉₁ coincident ou sont distants l'un de l'autre, de facon que le centre de rotation M₉₁ soit décalé par rapport au centre de rotation M₈₁ en direction de la cuisse et/ou latéralement vers l'intérieur médialement ou vers l'extérieur latéralement.

5. Articulation artificielle selon l'une des revendications 1 à 4, caractérisée en ce que les deux surfaces de glissement (13, 14) du corps de répartition de pression (10) sont de dimensions identiques.
